# EUROPEAN PATENT APPLICATION

(11) **EP 3 560 413 A1**
(43) Date of publication of application: **30.10.2019**
(21) Application number: 17884528.5
(22) Date of filing: 15.11.2017
(51) Int. Cl.: A61B 1/04, A61B 1/00

(54) **CAPSULE ENDOSCOPE APPARATUS FOR REPRODUCING 3D IMAGE, OPERATION METHOD FOR SAME CAPSULE ENDOSCOPE, RECEIVER FOR REPRODUCING 3D IMAGE IN ASSOCIATION WITH CAPSULE ENDOSCOPE, METHOD FOR REPRODUCING 3D IMAGE BY RECEIVER IN ASSOCIATION WITH CAPSULE ENDOSCOPE, AND CAPSULE ENDOSCOPE SYSTEM**

(30) Priority: 21.12.2016 KR 20160175579
(71) Applicant: Intromedic Co., Ltd., Seoul 08375 (KR)
(72) Inventor: OH, Jung Bum, Seoul 05509 (KR)
(74) Representative: Botti, Mario
(86) International application number: PCT/KR2017/012952
(87) International publication number: WO 2018/117427

(57) **Abstract**

An aspect of the present invention provides a capsule endoscope apparatus for reproducing a 3D image. The apparatus comprises: a first photographing unit for generating a first image by photographing a body part to be imaged; a second photographing unit for generating a second image by photographing the body part to be imaged; a control unit for simultaneously providing a trigger signal for synchronization to the first photographing unit and the second photographing unit and receiving a first image and a second image, which are simultaneously captured by the trigger signal, so as to generate a stereo image frame; and a transmission unit for transmitting the stereo image frame to a receiver.

## Description

### [Technical Field]

The present invention relates to a capsule endoscope apparatus and, more specifically, to a receiver for receiving an image captured by a capsule endoscope apparatus and performing image processing with respect to the captured image.

### [Background Art]

In order to acquire information on the inside of a human body, especially medical information, a method of inserting an endoscope attached to a cable through a subject's mouth or anus is used. According to this method, the endoscope is capable of being controlled using a cable formed of conducting wires or optical fibers and thus easy to secure data on the inside of the human body, but the subject has to endure pain. In addition, an organ like a small intestine is positioned far from the subject's mouth or anus and a diameter thereof is too small to examine the organ by the aforementioned endoscopy method.

In consideration of the above, a capsule endoscope is being used. If the subject swallows the capsule endoscope through the mouth, the capsule endoscope acquires necessary data in the human body through a camera or the like and transmits the acquired data to a receiver located outside the human body so that the data can be output.

However, the capsule endoscope provides just a 2D image using one image sensor it has a problem that it is difficult to determine a stereoscopic shape of an organ in the human body. In addition, since it is difficult to determine the stereoscopic shape, it is also difficult to determine an actual size of a specific abnormality in an image.

### [Disclosure]

### [Technical Problem]

One object in one general aspect of the present invention in order to solve the aforementioned problems is to provide a capsule endoscope apparatus supporting generation of a 3D image, and an operation method thereof.

In addition, another object in another aspect of the present invention is to provide a receiver for measuring an actual size of an object based on an image captured by a capsule endoscope, and an operation method of the receiver.

### [Technical Solution]

A capsule endoscope apparatus for reproducing a 3D image according to one aspect of the present invention in order to achieve the aforementioned objects includes: a first photographing unit configured to generate a first image by photographing a target body part of a human body; a second photographing unit configured to generate a second image by photographing the target body part; a control unit configured to simultaneously provide a trigger signal to the first photographing unit and the second photographing unit for synchronization, and generate a stereo image frame by receiving the first image and the second image simultaneously captured in response to the trigger signal; and a transmitting unit configured to transmit the stereo image frame to the receiver.

The control unit may generate a single stereo image frame from the first and second images simultaneously captured.

Camera calibration is performed in advance with respect to the first photographing unit and the second photographing unit.

The first image may be a left image of the target body part, and the second image may be a right image of the target body part.

An operation method of a capsule endoscope apparatus for reproducing a 3D image according to one aspect of the present invention in order to achieve the aforementioned objects includes: simultaneously providing a trigger signal for synchronization to a first photographing unit and a second photographing unit; generating, by the first and second photographing units, a first image and a second image by simultaneously photographing a target body part of a human body based on the trigger signal; generating a stereo image frame based on the first image and the second image that are simultaneously captured; and transmitting the stereo image frame to a receiver.

A receiver for reproducing a 3D image in association with a capsule endoscope apparatus according to one aspect of the present invention in order to achieve the aforementioned objects includes: a receiving unit configured to receive, from the capsule endoscope apparatus, a stereo image frame that is generated based on a first image and a second image generated by a first camera and a second camera of the capsule endoscope apparatus, by simultaneously photographing a target body part of a human body; a stereo image processing unit configured to generate a depth map by performing image processing with respect to the stereo image frame; and a Graphical User Interface (GUI) configured to render a 3D image based on the first image, the second image, and the depth map.

The image processing unit may include: a calibration unit configured to perform camera calibration based on the first image and the second image; a stereo rectification unit configured to generate row-aligned first image and a second image by performing stereo rectification based on information obtained through the calibration; a stereo matching unit configured to generate a disparity map by matching identical points in the row-aligned first image and second image; and a reprojection unit configured to generate a depth map by converting the disparity map into a distance.

The stereo image processing unit further comprises a quality improved image generation unit configured to improve a quality of the first image and the second image using a deblurring filter generated based on the depth map.

The quality improved image generation unit may improve an image quality by selecting an image having a better quality of the first image and the second image or by selecting a pixel having a better quality from the first image and the second image by each pixel.

The quality improved image generation unit may improve the quality of the first image and the second image using a Super-Resolution scheme.

The GUI may be further configured to reproduce a 3D image using the depth map based on a quality improved image.

The GUI may provide a function of measuring, using a parameter obtained through camera calibration, a size of a specific object selected by a user input in the quality improved image.

The GUI may display a 2D image and a 3D image together.

The GUI may include a 3D control interface for controlling the 3D image in a 3D space.

A method for reproducing a 3D image by a receiver in association with a capsule endoscope apparatus according to one aspect of the present invention to achieve the aforementioned objects includes: receiving, from a capsule endoscope apparatus, a stereo image frame generated by simultaneously photographing, by a first camera and a second camera of the capsule endoscope apparatus, a target body part of a human body; generating a depth map by performing image processing with respect to the stereo image frame; and rendering a 3D image based on the first image, the second image, and the depth map.

A capsule endoscope system for reproducing a 3D image according to one aspect of the present invention to achieve the aforementioned objects includes: a capsule endoscope apparatus configured to generate a first image and a second image by simultaneously photographing a target body part of a human body based on a trigger signal through a first photographing unit and a second photographing unit, generate a stereo image frame based on the first image and the second image, and transmit the stereo image frame to a receiver; and a receiver configured to receive the stereo image frame from the capsule endoscope apparatus, generate a depth map by performing image processing with respect to the received stereo image frame, and render a 3D image based on the first image, the second image, and the depth map.

A capsule endoscope system for reproducing a 3D image according to one aspect of the present invention to achieve the aforementioned objects includes: a capsule endoscope apparatus configured to generate a first image and a second image by simultaneously photographing a target body part of a human body based on a trigger signal through a first photographing unit and a second photographing unit, generate a stereo image frame based on the first image and the second image, and transmit the stereo image frame to a receiver; and a receiver configured to receive the stereo image frame from the capsule endoscope apparatus and transmits the stereo image fame to an image processing device; and the image processing device configured to generate a depth map by performing image processing with respect to the received stereo image frame, and render a 3D image based on the first image, the second image, and the depth map.

### [Advantageous Effects]

According to a capsule endoscope apparatus for reproducing a 3D image and a receiver for reproducing the 3D image in association with the capsule endoscope apparatus, there are advantages in that a 3D image in the shape of an actual stereoscopic shape of a target body part of a human body to increase accuracy in measuring the actual size of an object in the image and significantly improving the quality of the original image.

### [Description of Drawings]

FIG. 1 is a diagram showing a capsule endoscope system according to an embodiment of the present invention.
FIG. 2 is a flowchart schematically showing operations of a capsule endoscope apparatus and a receiver.
FIG. 3 is a block diagram schematically showing configuration of a capsule endoscope apparatus according to an embodiment of the present invention
FIG. 4 is a conceptual diagram for explanation of configuration of a stereo image frame.
FIG. 5 is a diagram schematically showing a receiver reproducing a 3D image in association with a capsule endoscope apparatus according to an embodiment of the present invention.
FIG. 6 is a detailed block diagram specifically showing a stereo image processing unit of the receiver in FIG.
FIG. 7 is a diagram showing a Graphical User Interface (GUI) simultaneously displaying a 2D image and a 3D image according to an embodiment of the present invention.
FIG. 8 is a diagram showing a GUI for measuring an actual size of an object according to another embodiment of the present invention.

### [Mode for Invention]

Since various modifications may be performed on the present invention and various embodiments of the present invention can be implemented, specific exemplary embodiments of the present invention will be described herein in detail with reference to the accompanying drawings.

However, the present invention will not be limited only to the specific exemplary embodiments of the present invention which are disclosed herein, and it should be understood that the scope and spirit of the present invention can be extended to all variations, equivalents, and replacements in addition to the appended drawings of the present invention.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the present invention. As used here, the term "and/or" includes any and all combinations of one or more of the associated listed items.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

The terminology used herein is for the purpose of describing particular embodiments only and is not intended to be limiting of the invention. As used herein, the singular forms are intended to include the plural forms as well, unless the context clearly indicates otherwise. It will be further understood that the terms "comprises/comprising" or "includes/including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined here.

Hereinafter, exemplary embodiments of the present invention will be described in more detail with reference to the accompanying drawings. In order to facilitate the general understanding of the present invention in describing the present invention, through the accompanying drawings, the same reference numerals will be used to describe the same components and an overlapped description of the same components will be omitted.

### Capsule Endoscope System

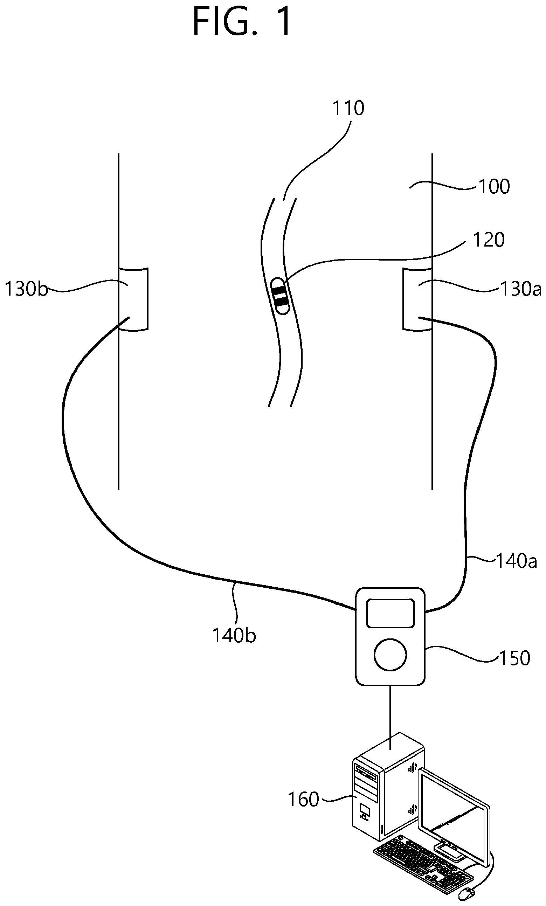

FIG. 1 is a diagram showing a capsule endoscope system according to an embodiment of the present invention. As shown in FIG. 1, the capsule endoscope system according to an embodiment of the present invention may include a capsule endoscope apparatus 120, a receiving electrode 130a and 130b, and a receiver 150.

Referring to FIG. 1, while the capsule endoscope apparatus 120 passes an organ 110, i.e., a small intestine and a large intestine, in a human body 100 of a subject, information on the corresponding organ is obtained. Information obtainable by the capsule endoscope apparatus 120 includes predetermined image information, acoustic information, and/or analysis information on a medium in the human body. In this case, the capsule endoscope apparatus 120 may generate a left image and a right image by photographing the organ 110 of the internal body with two or more cameras. The left image and the right image may be images that are photographed temporally at the same time.

The obtained information is converted into an electrical signal in the capsule endoscope apparatus 120 and sensed by the receiving electrode 130a and 130b attached to the body of the subject. The receiving electrode 130a and 130b delivers the received electrical signal to the receiver 150 through a conducting wire 140a and 140b.

Alternatively the obtained information may be converted into the electrical signal in the capsule endoscope apparatus 120 and delivered directly to the receiver 150 using a Radio Frequency (RF) or Human Body Communication (HBC). A method of using an RF is implemented in which the converted electrical signal is delivered to the receiver 150 using a frequency domain that is harmless to a human body. A method of using the HBC is implemented in a manner in which, when an electrode provided on an outer surface of the capsule endoscope apparatus 120 is brought into contact with the human body due to peristalsis of the organ 110 in the human body 100, a current is generated and the converted electrical signal is delivered to the receiver 150 using the current.

The receiver 150 configured to receive a left image and a right image from the capsule endoscope apparatus 120 may generate a 3D image through stereo image processing and reproduce the 3D image via a Graphic User Interface (GUI). Alternatively, the receiver 150 may generate a depth map required to generate a 3D image, and perform an image quality improvement process.

According to an embodiment of the present invention, the receiver 150 may simply receive a left image and a right image and transmits the received images to a different image processing apparatus 160 (e.g., a different PC, a laptop, a smart phone, or any other device wiredly or wirelessly connected with the receiver 150) so as to allow the separate image processing apparatus 160 to perform procedures subsequent to the stereo image processing, so that a 3D image is reproduced.

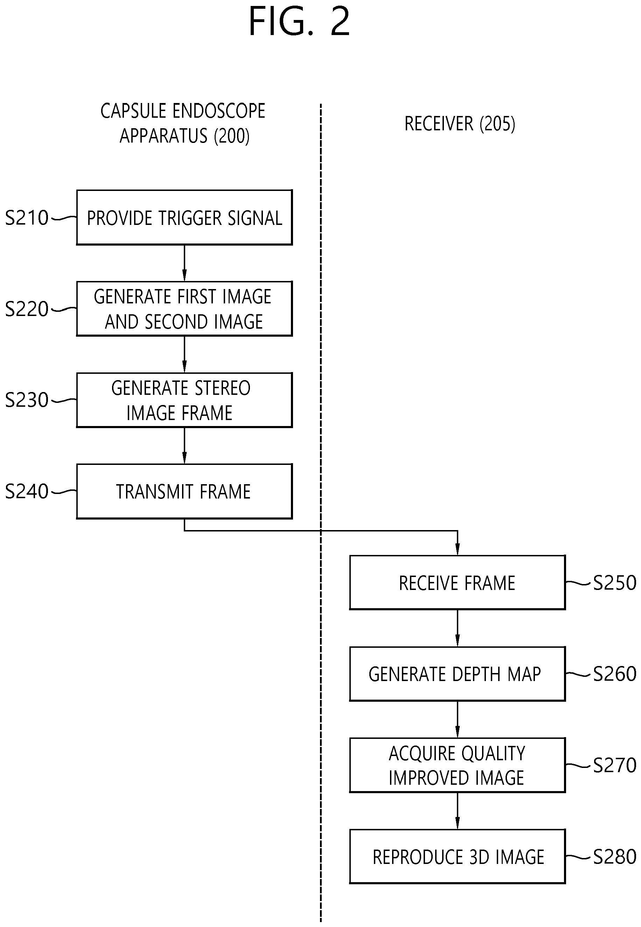

FIG. 2 is a flowchart schematically showing operations of a capsule endoscope apparatus and a receiver.

Referring to FIG. 2, a control unit of a capsule endoscope apparatus 200 provides a trigger signal so as to allow two or more photographing units to simultaneously photograph a specific body part (S210). In this case, it is preferable that the trigger signal simultaneously arrives at the two or more photographing units within an error of 10ms.

The two or more photographing units having received the trigger signal generate a first image and a second image (S220) by photographing the specific body part simultaneously. In this case, the first image is a left image (L) of the specific body part, and the second image may be a right image (R) of the specific body part. If there is an additional photographing unit, a third image may be generated, and the third image may be a middle image.

The control unit of the capsule endoscope apparatus 200 receives data related to the first image and the second image, and generates one stereo image frame (S230) by inserting a synchronization signal. That is, the simultaneously photographed first and second images are used together with time-related information to generate one frame.

Then, the generated stereo image frame is transmitted to a receiver 205 (S240).

The receiver 205 receives the stereo image frame from the capsule endoscope apparatus 200 (S250), and extracts data related to the first image and the second image from the corresponding frame.

Then, the receiver 205 generates a depth map (S260) by performing calibration, stereo rectification, stereo matching, and reprojection on the first and second images. In this case, two quality-improved images may be obtained (S270) by using a deblurring filter generated based on a depth map for the left/right original images and a Super-Resolution scheme.

Then, a 3D image may be generated based on the quality-improved image and the depth map and reproduced via a Graphic User Interface (GUI) (S280). In this case, a size of a specific object (e.g., a tumor in an organ) in the image may be calculated precisely.

### Capsule endoscope apparatus

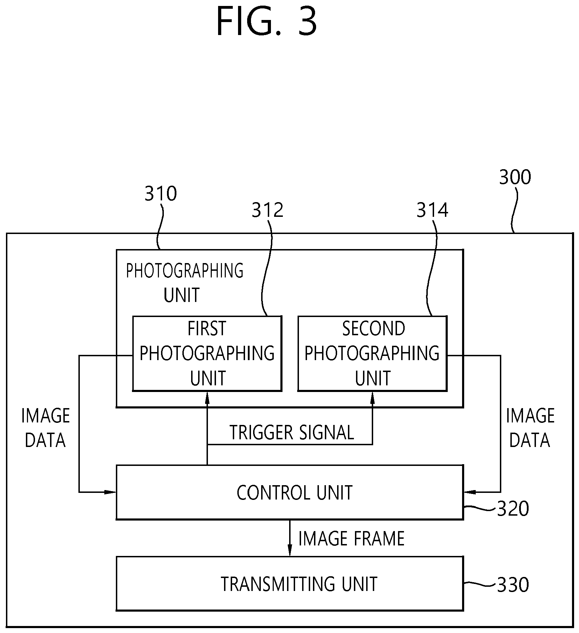

FIG. 3 is a block diagram schematically showing configuration of a capsule endoscope apparatus according to an embodiment of the present invention. As shown in FIG. 3, a capsule endoscope apparatus 300 according to an embodiment of the present invention may include a photographing unit 310, a control unit 320, and a transmitting unit 330.

Referring to FIG. 3, the capsule endoscope apparatus 300 may have a smooth cylindrical structure to be used without damaging a human body, and one end and/or the other end of the capsule endoscope apparatus 300 may be in a dorm shape.

The photographing unit 310 is an element configured to obtain image data by photographing an internal organ in a human body. According to an embodiment of the present invention, the photographing unit 310 includes a first photographing unit 312 and a second photographing unit 314. In some cases, two or more photographing units (e.g., three photographing units) may be included. The photographing units may be formed at one end of the capsule endoscope apparatus 300 or may be formed at both ends thereof.

The photographing unit 310 may include a lighting unit (not shown). When the inside of the human body is lighted by the lighting unit, the first and second photographing units 312 and 314 photograph an image of a lighted part. The lighting unit includes one or more light emitting devices, such as an LED. The lighting unit may be disposed around the first and second photographing units 312 and 314 and light up a part to be photographed by the first and second photographing units 312 and 314.

The lighting unit may use a light emitting device of a specific wavelength according to which body part to be examined, for example, if presence of cancer is to be examined, and, if so, what kind of cancer is to be examined, which body part is to be examined, if a state of a tissue is to be examined, etc. In addition, according to which device is used as a photographing device, a light emitting device adequate for the photographing device may be used.

The first and second photographing units 312 and 314 includes a photographing device, such as a Complementary Metal-Oxide Semiconductor (CMOS) image sensor, a Charge-Coupled Device (CCD) image sensor, etc. An image acquired by the photographing device may be converted into electrical data or an electrical signal.

Since the first and second photographing units 312 and 314 are elements configured to generate a 3D image, it is preferable that camera calibration is performed in advance. That is, in order to perform photographing in association with calibration in the receiver, mechanism of a camera, such as lenses used in the first and second photographing units 312 and 314, a distance between a lens and an image sensor, and an angle formed by a lens and an image sensor, may be calibrated in advance as defined by a user. In this case, information related to the calibration may be shared with the receiver.

In addition, the first and second photographing units 312 and 314 may photograph the same target body part in response to a trigger signal from the controller 320. However, the first and second photographing units 312 and 314 may view the target body part at different angles and thus acquire a left image and a right image with respect to one target body part. Since the first and second photographing units 312 and 314 simultaneously receives a trigger signal, the first and second photographing units 312 and 314 may photograph a target body part at the same point in time, and thus, a left image and a right image of the target body part at the same point in time may be obtained. Synchronization of the left image and the right image is a very critical issue to configure a 3D image, and hence, it is preferable that the left image and the right image photographed at the same point in time are handled as one package.

The control unit 320 is an element configured to control the first and second photographing units 312 and 314 and perform image processing with respect to a left image and a right image photographed by the first and second photographing units 312 and 314. In order to synchronize the left image and the right image, the control unit 320 provides a trigger signal to the first and second photographing units 312 and 314 at the same point in time. As described above, it is preferable that the trigger signal arrives at the first and second photographing units 312 and 314 within an error of 10ms. The control unit 320 simultaneously provides the trigger signal to the first and second photographing units 312 and 314 with reference to a clock frequency.

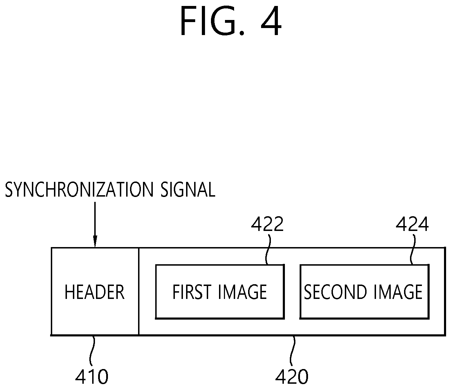

The controller 320 receives the left image and the right image simultaneously photographed in response to the trigger signal, and encodes the left image and the right image. Then, the controller 320 generates one frame packet by binding the encoded left and right images. Generating a frame packet will be described in more detail with reference to FIG. 4.

The transmitting unit 330 transmits the image fane, generated by the controller 320, to an external device, i.e., a receiver 150 (see FIG. 1). For example, the transmitting unit 330 may transmit image data to an external device through RF communication or HBC, and may transit other information (e.g., acoustic information, tissue information, PH information, temperature information, electrical impedance information, etc.) in addition to impressed image data.

FIG. 4 is a conceptual diagram for explanation of configuration of a stereo image frame.

Referring to FIG. 4, a stereo image frame may be composed of a header 410 and a payload 420.

First, an encoded first image 422 and an encoded second image 424 may be included in the payload 420. In this case, the first image 422 may be a left image of a specific target body part, and the second image 424 may be a right image of the specific target body part. Since the two images 422 and 424 are images photographed at the same point in time, it is preferable that the two images 422 and 424 are combined together as the payload 420 of one frame and transmitted.

The header 410 may include synchronization information of the two images 422 and 424. The synchronization information may include metadata information related to a photographing time of the two images 422 and 424. Information included in the header 410 may include information a size of a stereo image frame, time of when the images are acquired, an encoding-related factor, a frame number of the corresponding image frame, etc.

One package of image data or a plurality of packages of image frames may be included in the payload 420. However, it is preferable that image data on the basis of a package unit photographed at the same point in time is included in the payload 420.

In the case of transmitting images in plural packages, metadata related to image frames in N number (N is a natural number equal to or greater than 2) is included in the frame header 410. In this case, metadata information in plural related to each package may be included. The metadata information includes synchronization information of each package, and other information included in the metadata information is similar as in the aforementioned embodiment. In addition, data on first images 422 and second images 424 in N number of packages may be continuously stored in the payload 420.

### Receiver in association with Capsule Endoscope Apparatus

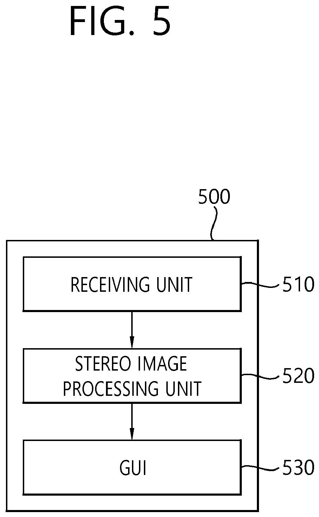

FIG. 5 is a diagram schematically showing a receiver reproducing a 3D image in association with a capsule endoscope apparatus according to an embodiment of the present invention. As shown in FIG. 5, a receiver 500 according to an embodiment of the present invention may include a receiving unit 510, a stereo image processing unit 520, and a Graphic User Interface (GUI) 530.

Referring to FIG. 5, the receiver 510 receives a stereo image frame transmitted from a capsule endoscope apparatus. Data in the received frame is encoded data including a package of a left image and a right image photographed with respect to the same subject, and may include other information (e.g., acoustic information, tissue information, PH information, temperature information, electrical impedance information, etc.). Meanwhile, the receiving unit 510 may be configured as an electrode or a pad attached to the body of a subject for whom the capsule endoscope apparatus is used.

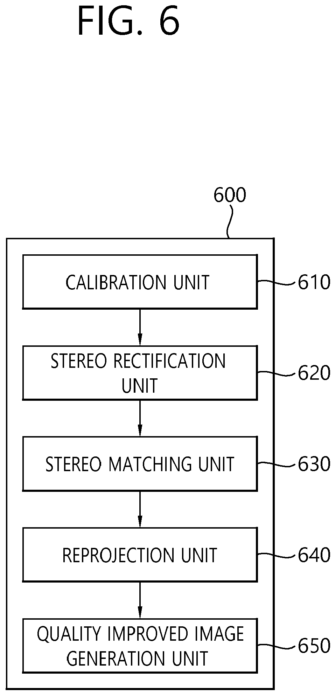

The stereo image processing unit 520 extracts an image data package and synchronization information included in the stereo image frame received by the receiving unit 510, decodes the extracted image data package and the extracted synchronization information, and generate a depth map by performing stereo image processing. Then, image quality improvement may be performed. The stereo image processing unit 520 may generate the depth map by performing calibration, stereo rectification, stereo matching, and reprojection. In addition, an image with a further improved quality may be generated by applying a deblurring filter, generated based on the depth map, and a Super-Resolution scheme to the left/right original images. The stereo image processing procedure will be described in more detail with reference to FIG. 6.

The GUI 530 may generate a 3D image based on a left/right original image and a depth map. However, it is more preferable that a 3D image is reproduced based on a quality-improved image and a depth map. In addition, a user image may be received while an image is displayed, so that an actual size of an object indicated by a user may be calculated based on camera calibration information and displayed.

According to an embodiment of the present invention, the receiving unit 510 only may be included as an element of the receiver 500, and the stereo image processing unit 520 and the GUI 530 may be implemented as separate image processing devices. Alternatively, the receiver 500 may include the receiving unit 510 and the stereo image processing unit 520, and the GUI 530 may be implemented as a separate image processing device.

FIG. 6 is a detailed block diagram specifically showing a stereo image processing unit of the receiver in FIG. 5. As shown in FIG. 6, a stereo image processing unit 600 according to an embodiment of the present invention may include a calibration unit 610, a stereo rectification unit 620, a stereo matching unit 630, a reprojection unit 640, and an image improved image generation unit 650.

Referring to FIG. 6, although not illustrated therein, a received stereo image frame is pared to extract a package of a first image (a left image) and a second image (a right image). In this case, synchronization information is parsed to identify a point in time when the images were photographed, and image processing is performed to bind a left image and a right image as one package. First, an encoded left image and an encoded right image are decoded, and a 3D vision-related procedure is performed.

With respect to the left image and the right image, the calibration unit 610 calculates a conversion relation between 3D space coordinates and 2D image coordinates, or a parameter indicative of this conversion relation.

The calibration unit 610 calculates an intrinsic parameter and extrinsic parameter based on M number of chess board images (M is a natural number) photographed by two cameras in the capsule endoscope apparatus.

The intrinsic parameter may use a focal distance, a principal point, a skew coefficient, etc. The focal distance refers to a distance between a lens center and an image sensor, which can be calculated on a pixel unit basis. The principal point may indicates image coordinates of a foot of perpendicular from the center of a camera lens, that is, a pin hole, to the image sensor, and the principal point may be calculated on a pixel unit basis. The skew coefficient indicates the degree of skewness of a cell array of the image sensor and the Y-axis.

The extrinsic parameter is a parameter indicative of a conversion relation between a camera coordinate system and a word coordinate system, which can be expressed as rotation and translation of the two coordinate systems.

In addition to the intrinsic and extrinsic parameters, the calibration unit 610 may calculate a distortion parameter for each camera, an essential matrix representing a positional relationship between calibrated cameras, and a fundamental matrix representing information related to corresponding points between a left image and a right image.

The stereo rectification unit 620 generates row-aligned images based on image acquired by the calibration unit 610 (e.g., an intrinsic parameter, an extrinsic parameter, etc.). The stereo rectification unit 620 may rectify a left image and a right image based on calibration information so that the images are rectified as if the two images are photographed by one row-aligned camera.

The stereo matching unit 630 may find identical points in the left image and the right image and match the identical points. The stereo matching is allowed only in a portion in which the two images overlap. The stereo matching unit 630 performs pre-processing to normalize brightness of an image and improves texture. Then, the matching is performed by performing post-processing such that a corresponding point is discovered by moving a SAD (Sum of Absolut Difference: a sum of absolute values of differences between pixels values within a given window) window, and a wrong corresponding point is removed. The stereo matching unit 630 may generate a disparity map by matching corresponding points. In addition, a merged image may be generated using corresponding point information about a left image and a right image and baseline separation information about two cameras. In this case, radial distortion and tangential distortion of a lens may be removed mathematically.

The reprojection unit 640 generates a depth map by converting a disparity map into a distance using trigonometry. After the depth map is generated, a depth of each point in an original image (whether a corresponding protrudes forward or is sunk backward) may be clearly aware of, and thus, it is possible to know a stereoscopic shape or an exact size thereof using the depth map.

The quality improved image generator 650 selects a better quality image out of a left image and a right image. In this case, both the two original image may be improved in quality by selecting one of the images or by selecting a pixel of an image having a better quality in pixels when corresponding points are known. Then, the reprojection unit 640 improves quality of one image or two images through a deblurring filter that uses the generated depth map. That is, according to an embodiment of the present invention, the deblurring filter is generated using the depth filter, and the deblurring filter is used to improve image quality. Then, a Super-Resolution technique is implemented, thereby completing improving image quality.

Taken as a whole, the stereo image processing unit 600 receives a left image and a right image, and outputs a depth map and a quality-improved image via a user interface.

When a request for calculation of a size of an object in an image is received via the GUI (which is, for example, implemented as a keyboard, a mouse, a touch screen, a display, etc.)The stereo image processing unit 600 may calculate an actual size of the object by combining pixel data of the object and left/right camera calibration information obtained through camera calibration, and provide the calculated actual size of the object via the GUI.

### Graphic User Interface of Receiver

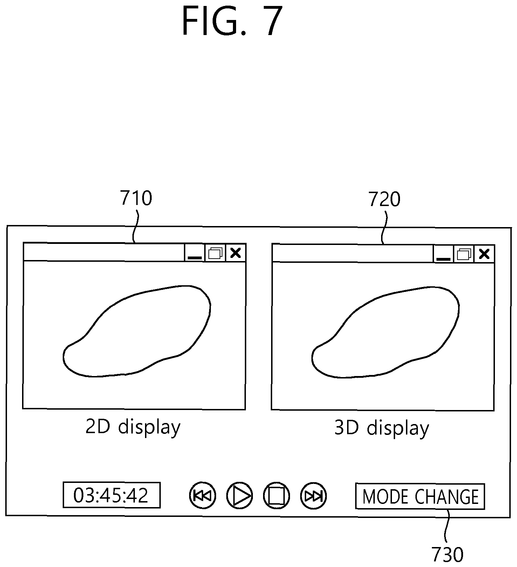

FIG. 7 is a diagram showing a Graphical User Interface (GUI) simultaneously displaying a 2D image and a 3D image according to an embodiment of the present invention.

Referring to FIG. 7, a 2D image 710 and a 3D image 720 may be displayed simultaneously. The 2D image 710 and the 3D image 720 may be displayed on one window or may be displayed on different windows. In addition, the 2D image 710 and the 3D image 720 may be synchronized so that an image at the same point in time may be reproduced. In contrast, images at different point in time may be displayed on one screen. As the 2D image 710, a quality improved image may be used.

The GUI according to an embodiment of the present invention may provide various modes, and the mode may change via a mode change button 730. The modes may include a mode in which both a 2D left image and a 2D right image are provided, a mode in which one 2D image only is provided, a mode in which one 3D image only is provided, and a mode in which a 2D image and a 3D image are provided together. A user is capable of changing an image display mode with simple manipulation. For example, when a 3D image is requested while a 2D image is reproduced, it may be set to display the 2D image and the 3D image on the same screen.

In addition, according to an embodiment of the present invention, when 3D image is displayed, a 3D control GUI for freely controlling the 3D image may be provided.

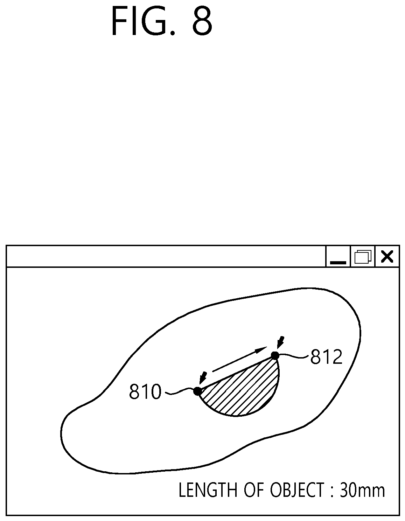

FIG. 8 is a diagram showing a GUI for measuring an actual size of an object according to another embodiment of the present invention.

Referring to FIG. 8, in correspondence to the fact that a specific pixel in an image in a single camera corresponds to an actual size of several mm, a method of identifying an object in the image on a pixel unit basis is used, but this method is not accurate because stereoscopic properties are not taken into consideration.

In a 3D image captured by a stereo camera according to the present invention, a pixel may be converted into a specific length unit (e.g., a unit of mm) with an intrinsic parameter obtained from camera calibration.

For example, if there is a portion such as a tumor, an actual size of which a user wants to check, in a quality improved image, the portion to be checked in an image using a click or the like, for example, a portion from a point 810 to a point 820, is designated clearly, and an actual pixel corresponding to the designated portion is identified. Then, a distance between pixels and an intrinsic parameter acquired through camera calibration are input to an equation related to 3D vision to be converted into an accurate actual size. A size of the converted size may be displayed on a screen together with a 3D image. A function of converting into an actual size may be applied to detecting a serious abnormality in a human body.

While the present invention has been described with respect to the accompanying drawings and aforementioned embodiments, it should not understood that the scope of the present invention is not limited by the accompanying drawings and the embodiments, and it will be apparent to those skilled in the art that various changes and modifications may be made without departing from the spirit and scope of the invention as defined in the following claims.

## Claims

1. A capsule endoscope apparatus for reproducing a 3D image, comprising:
a first photographing unit configured to generate a first image by photographing a target body part of a human body;
a second photographing unit configured to generate a second image by photographing the target body part;
a control unit configured to simultaneously provide a trigger signal to the first photographing unit and the second photographing unit for synchronization, and generate a stereo image frame by receiving the first image and the second image simultaneously captured in response to the trigger signal; and
a transmitting unit configured to transmit the stereo image frame to the receiver.

2. The capsule endoscope apparatus of claim 1, wherein the control unit generates a single stereo image frame from the first and second images simultaneously captured.

3. The capsule endoscope apparatus of claim 1, wherein camera calibration is performed in advance with respect to the first photographing unit and the second photographing unit.

4. The capsule endoscope apparatus of claim 1, wherein the first image is a left image for the target body part, and the second image is a right image for the target body part.

5. An operation method of a capsule endoscope apparatus for reproducing a 3D image, the method comprising:
simultaneously providing a trigger signal for synchronization to a first photographing unit and a second photographing unit;
generating, by the first and second photographing units, a first image and a second image by simultaneously photographing a target body part of a human body based on the trigger signal;
generating a stereo image frame based on the first image and the second image that are simultaneously captured; and
transmitting the stereo image frame to a receiver.

6. A receiver for reproducing a 3D image in association with a capsule endoscope apparatus, the receiver comprising:
a receiving unit configured to receive, from the capsule endoscope apparatus, a stereo image frame that is generated based on a first image and a second image generated by a first camera and a second camera of the capsule endoscope apparatus, by simultaneously photographing a target body part of a human body;
a stereo image processing unit configured to generate a depth map by performing image processing with respect to the stereo image frame; and
a Graphical User Interface (GUI) configured to render a 3D image based on the first image, the second image, and the depth map.

7. The method of claim 6, wherein the image processing unit comprises:
a calibration unit configured to perform camera calibration based on the first image and the second image;
a stereo rectification unit configured to generate row-aligned first image and second image by performing stereo rectification based on information obtained through the calibration;
a stereo matching unit configured to generate a disparity map by matching identical points in the row-aligned first image and second image; and
a reprojection unit configured to generate a depth map by converting the disparity map into a distance.

8. The receiver of claim 7, wherein the stereo image processing unit further comprises a quality improved image generation unit configured to improve a quality of the first image and the second image using a deblurring filter generated based on the depth map.

9. The receiver of claim 8, wherein the quality improved image generation unit improves an image quality by selecting one image having a better quality of the first image and the second image or by selecting a pixel having a better quality from the first image and the second image by each pixel.

10. The receiver of claim 9, wherein the quality improved image generation unit improves the quality of the first image and the second image using a Super-Resolution scheme.

11. The receiver of claim 10, wherein the GUI is further configured to reproduce a 3D image using the depth map based on a quality improved image.

12. The receiver of claim 10, wherein the GUI is provides a function of measuring, using a parameter obtained through camera calibration, a size of a specific object selected by a user input in the quality improved image.

13. The receiver of claim 6, wherein the GUI displays a 2D image and a 3D image together.

14. The receiver of claim 6, wherein the GUI comprises a 3D control interface for controlling the 3D image in a 3D space.

15. A method for reproducing a 3D image by a receiver in association with a capsule endoscope apparatus, the method comprising:
receiving, from a capsule endoscope apparatus, a stereo image frame generated by simultaneously photographing, by a first camera and a second camera of the capsule endoscope apparatus, a target body part of a human body;
generating a depth map by performing image processing with respect to the stereo image frame; and
rendering a 3D image based on the first image, the second image, and the depth map.

16. A capsule endoscope system for reproducing a 3D image, the system comprising:
a capsule endoscope apparatus configured to generate a first image and a second image by simultaneously photographing a target body part of a human body based on a trigger signal through a first photographing unit and a second photographing unit, generate a stereo image frame based on the first image and the second image, and transmit the stereo image frame to a receiver; and
a receiver configured to receive the stereo image frame from the capsule endoscope apparatus, generate a depth map by performing image processing with respect to the received stereo image frame, and render a 3D image based on the first image, the second image, and the depth map.

17. A capsule endoscope system for reproducing a 3D image, the system comprising:
a capsule endoscope apparatus configured to generate a first image and a second image by simultaneously photographing a target body part of a human body based on a trigger signal through a first photographing unit and a second photographing unit, generate a stereo image frame based on the first image and the second image, and transmit the stereo image frame to a receiver; and
a receiver configured to receive the stereo image frame from the capsule endoscope apparatus and transmits the stereo image fame to an image processing device; and
the image processing device configured to generate a depth map by performing image processing with respect to the received stereo image frame, and render a 3D image based on the first image, the second image, and the depth map.
